# EUROPEAN PATENT APPLICATION

(11) **EP 3 466 336 A1**
(43) Date of publication of application: **10.04.2019**
(21) Application number: 17820107.5
(22) Date of filing: 26.06.2017
(51) Int. Cl.: A61B 5/08, A61B 5/00

(54) **BREATHING ABNORMALITY DETECTION DEVICE AND BREATHING ABNORMALITY DETECTION METHOD**

(30) Priority: 29.06.2016 JP 2016128650
(71) Applicant: Konica Minolta, Inc., Tokyo 100-7015 (JP)
(72) Inventor: OKADA, Masakazu, Tokyo 100-7015 (JP); MASAZUMI, Naoki, Tokyo 100-7015 (JP)
(74) Representative: Hoffmann Eitle
(86) International application number: PCT/JP2017/023433
(87) International publication number: WO 2018/003752

(57) **Abstract**

An acquisition unit acquires a biological signal of a monitor subject serving as a target to be monitored. A generation unit generates a respiratory signal indicating a respiratory state of the monitor subject by using the biological signal acquired by the acquisition unit. A selection unit selects one or more respiratory waveform patterns from among a plurality of respiratory waveform patterns indicating a plurality of types of respiratory abnormalities by using a predetermined indicator serving as an indicator indicating a health condition of the monitor subject. A determination unit determines whether a waveform of the respiratory signal generated by the generation unit corresponds to one of the one or more respiratory waveform patterns selected by the selection unit, by using the waveform of the respiratory signal generated by the generation unit and the one or more respiratory waveform patterns selected by the selection unit.

## Description

### Technical Field

The present invention defines, for example, a person who needs nursing or a person who needs care (hereinafter referred to as a person requiring nursing care or the like) as a monitor subject, and relates to a technique to monitor a respiratory abnormality of the monitor subject.

### Background Art

Japan has become an aging society, more particularly, a super aging society in which an aging ratio, which is a proportion of a population aged 65 and over in the total population, exceeds 21%, through improvement in a living standard that attends postwar rapid economic growth, improvement in a hygiene environment, advancement of a medical standard, and other reasons. According to statistics in September, 2013, the aged population is about 31,860,000, the aging ratio is 25.0%, and one in four people is an aged person. There is an estimate that the aged population will be about 37,410,000 and one in three people will be an aged person in 2035 (Statistics Bureau, Ministry of Internal Affairs and Communications, Japan). In such an aging society, an increase in the number of persons requiring nursing care or the like due to illness, injury, advanced age, and the like is more expected than in a normal society that is not an aging society.

Such a person requiring nursing care or the like enters a hospital or a facility such as a welfare facility for the aged (a short-stay facility for the aged, a nursing home for the aged, a special nursing home for the aged, and the like under the Japanese statute), and receives nursing or care there. In such a hospital or a facility such as a welfare facility for the aged, in order for a person requiring nursing care or the like to stay comfortably without anxiety, for example, a person who nurses or cares for a person requiring nursing care or the like, such as a nurse and a caregiver (hereinafter referred to as a nurse or the like) confirms safety of the person by patrolling regularly. However, since the number of nurses or the like is smaller in a time period of semi-night shift or night shift than in a time period of day shift, resulting in an increase in a work load imposed on each person, reduction in the work load is required. Therefore, in recent years, a monitor-subject monitoring device for monitoring a person requiring nursing care or the like as a monitor subject has been studied and developed.

Examples of a technique regarding monitoring a monitor subject include a device for detecting a respiratory abnormality of the monitor subject, and a system for remotely caring for the monitor subject. As a former example, Patent Literature 1 discloses a device that generates movement amount waveform data by irradiating a sleeping monitor subject with an infrared ray and performing image processing on images (frames) captured continuously, the device detecting a respiratory abnormality of the monitor subject by using this movement amount waveform data. As a latter example, Patent Literature 2 discloses a remote care system that remotely cares for and monitors each monitor subject, and collects information about entering and leaving a room, rest room use information, information about falling from a bed, a heart rate, a respiratory rate, time to go to bed, time to leave bed, and the like about each monitor subject, analyzes the information, and determines staff placement based on an analysis result.

A respiratory waveform pattern differs depending on a type of respiratory abnormality (sleep apnea syndrome, bradypnea, tachypnea, and the like). There is a technique to determine the type of respiratory abnormality using this respiratory waveform pattern. This technique prepares a plurality of types of respiratory waveform patterns indicating respiratory abnormalities in advance (respiratory waveform pattern of sleep apnea syndrome, respiratory waveform pattern of bradypnea, respiratory waveform pattern of tachypnea, and the like), determines whether a waveform of a respiratory signal of the monitor subject agrees with one of the respiratory waveform patterns prepared in advance by using pattern matching. When the waveform of the respiratory signal agrees with one of the respiratory waveform patterns, this technique determines the respiratory abnormality of the type indicated by the respiratory waveform pattern. However, the present inventor has found out that as the number of types of respiratory waveform patterns indicating respiratory abnormalities increases, a possibility of performing erroneous determination increases in pattern matching. The erroneous determination includes determining normal respiration as a respiratory abnormality, and determining the type of respiratory abnormality by mistake. The former causes a monitor to run unnecessarily, whereas the latter causes a delay in dealing with the respiratory abnormality.

### Citation List

### Patent Literature

Patent Literature 1: Japanese Patent Application Laid-Open No. 2002-175582
Patent Literature 2: Japanese Patent Application Laid-Open No. 2001-258859

### Summary of Invention

An object of the present invention is to provide a respiratory abnormality detection device that can improve determination accuracy whether respiration is abnormal and determination accuracy of the type of respiratory abnormality, and a respiratory abnormality detection method.

A respiratory abnormality detection device according to a first aspect of the present invention includes an acquisition unit, a generation unit, a selection unit, and a determination unit. The acquisition unit acquires a biological signal of a monitor subject serving as a target to be monitored. The generation unit generates a respiratory signal indicating a respiratory state of the monitor subject by using the biological signal acquired by the acquisition unit. The selection unit selects one or more respiratory waveform patterns from among a plurality of respiratory waveform patterns indicating a plurality of types of respiratory abnormalities by using a predetermined indicator serving as an indicator indicating a health condition of the monitor subject. The determination unit determines whether a waveform of the respiratory signal generated by the generation unit corresponds to one of the one or more respiratory waveform patterns selected by the selection unit, by using the waveform of the respiratory signal generated by the generation unit and the one or more respiratory waveform patterns selected by the selection unit.

The above as well as additional objects, features, and advantages of the present invention will become apparent from the following detailed description and the accompanying drawings.

### Brief Description of Drawings

FIG. 1 is an explanatory diagram describing a configuration of a monitor-subject monitoring system according to an embodiment.
FIG. 2 is a schematic view showing a living room in which a sensor device is installed.
FIG. 3 is a block diagram showing a configuration of the sensor device included in the monitor-subject monitoring system according to the embodiment.
FIG. 4 is an explanatory diagram describing various types of respiratory waveform patterns.
FIG. 5 is a block diagram showing a configuration of a portable terminal device included in the monitor-subject monitoring system according to the embodiment.
FIG. 6 is a block diagram showing a configuration of a management server device included in the monitor-subject monitoring system according to the embodiment.
FIG. 7 is a flowchart describing respiratory abnormality detection and image recording to be performed by the monitor-subject monitoring system according to the embodiment.
FIG. 8 is a block diagram showing a configuration of a sensor device included in modification 1.
FIG. 9 is a flowchart describing respiratory abnormality detection and image recording to be performed by a monitor-subject monitoring system according to modification 1.
FIG. 10 is a block diagram showing a configuration of a sensor device included in modification 2.
FIG. 11 is a block diagram showing a configuration of a management server device included in modification 2.

### Description of Embodiment

An embodiment of the present invention will be described in detail below with reference to the drawings. In each of the drawings, a component with the same reference sign indicates the same component, and descriptions of the component already described will be omitted. In this specification, when a component is generically called, the component is denoted with a reference sign with a subscript omitted, whereas when an individual component is referred to, the component is denoted with a reference sign with a subscript attached.

A monitor-subj ect monitoring system according to the embodiment is a system that monitors a monitor subject who is a target to be monitored (in other words, a person to be watched who is a target to be watched) by using a plurality of devices. The monitor-subject monitoring system according to the embodiment includes: a terminal device; and a monitor-subject monitoring device communicatively connected to the terminal device, the monitor-subject monitoring device detecting a predetermined event (phenomenon) related to the monitor subject and notifying the terminal device of the event. Although the monitor-subject monitoring device may be integrally formed of one device, in this specification, the monitor-subject monitoring device is formed of two types of separate devices by including a sensor device and a management server device communicatively connected to the sensor device and the terminal device. The sensor device detects the predetermined event related to the monitor subject, and notifies (informs, transmits) the management server device. Upon receipt of the notification from the sensor device, the management server device manages the notified event, and re-notifies (re-informs, re-transmits), of the event, the predetermined terminal device associated with the sensor device. The terminal device may be one type of device, but in this specification, the terminal device is two types of devices: fixed terminal device and portable terminal device. A major difference between the fixed terminal device and the portable terminal device is that the fixed terminal device is fixedly operated, whereas the portable terminal device is carried and operated by, for example, an observer NS (user) such as a nurse and a caregiver. Since the fixed terminal device and the portable terminal device are generally similar, the portable terminal device will be mainly described below.

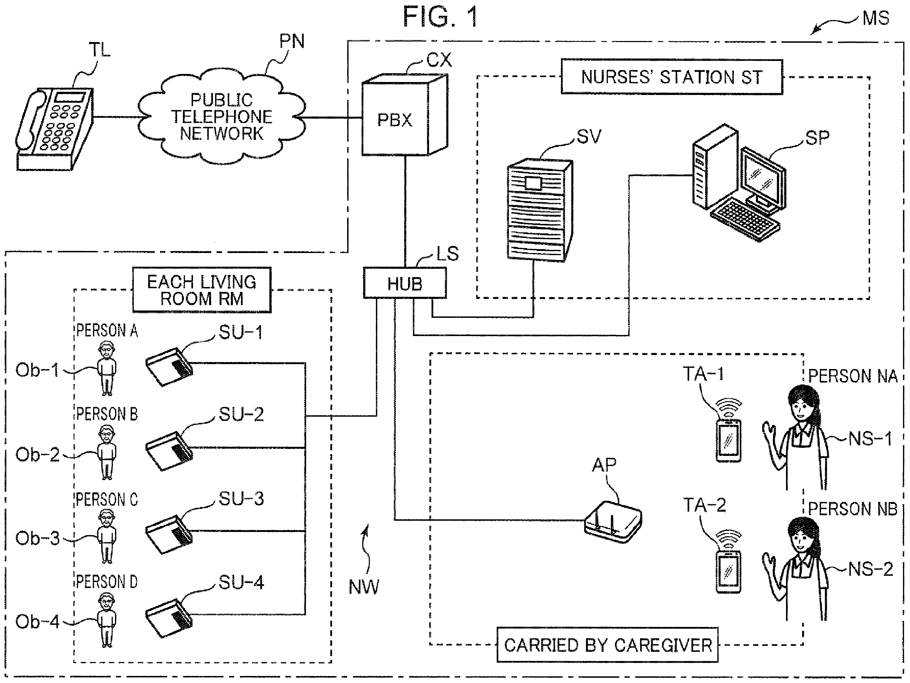

FIG. 1 is an explanatory diagram describing a configuration of a monitor-subject monitoring system MS according to the embodiment. More specifically, the monitor-subject monitoring system MS includes, for example, one or more sensor devices SU (SU-1 to SU-4), a management server device SV, a fixed terminal device SP, one or more portable terminal devices TA (TA-1 and TA-2), and a private branch exchange (PBX) CX. These devices are communicatively connected via a network (network or communication line) NW, such as a local area network (LAN) with a cable or wirelessly. The network NW may also include a relay for relaying a communication signal such as, for example, a repeater, a bridge, and a router. In the example shown in FIG. 1, the plurality of sensor devices SU-1 to SU-4, the management server device SV, the fixed terminal device SP, the plurality of portable terminal devices TA-1 and TA-2, and the private branch exchange CX are communicatively connected to one another via a LAN (for example, LAN in compliance with the IEEE 802.11 standard or the like) NW in which cable and wireless networks are mixed including a line concentrator (hub, HUB) LS of an L2 switch and an access point AP. More particularly, the plurality of sensor devices SU-1 to SU-4, the management server device SV, the fixed terminal device SP, and the private branch exchange CX are connected to the line concentrator LS, and the plurality of portable terminal devices TA-1 and TA-2 are connected to the line concentrator LS via the access point AP. The network NW constructs a so-called intranet by using Internet protocol suites, such as transmission control protocol (TCP) and Internet protocol (IP). The private branch exchange CX is connected to a telephone TL via a public telephone network PN.

The monitor-subject monitoring system MS is installed in an appropriate place according to a monitor subject Ob. The monitor subject Ob is, for example, a person who needs nursing care due to illness, injury, or other reasons, a person who needs care due to a decrease in physical ability or other reasons, a person living alone, and the like. Particularly, from a viewpoint of enabling early detection and early taking measures, the monitor subject Ob is preferably, for example, a person who needs detection of a predetermined inconvenient event such as an abnormal condition when the event occurs in the person. Therefore, the monitor-subject monitoring system MS is suitably installed in buildings such as a hospital, a welfare facility for the aged, and a dwelling unit according to the type of the monitor subject Ob. In the example shown in FIG. 1, the monitor-subject monitoring system MS is installed in a plurality of living rooms RM in which a plurality of monitor subjects Ob reside, and a building of a care home including a plurality of rooms such as a nurses' station.

The sensor device SU is a device having a communication function and the like for communicating with other devices SV, SP, and TA via the network NW. The device detects a predetermined event related to the monitor subject Ob and notifies the management server device SV of this detected event. The device performs a voice call with the terminal devices SP and TA. The device generates an image including a moving image and distributes the moving image to the terminal devices SP and TA. The predetermined event preferably includes an event that needs to be addressed.

FIG. 1 shows four first to fourth sensor devices SU-1 to SU-4 as one example. The first sensor device SU-1 is installed in a living room RM-1 (not shown) of Person A Ob-1 who is one of the monitor subjects Ob. The second sensor device SU-2 is installed in a living room RM-2 (not shown) of Person B Ob-2 who is one of the monitor subjects Ob. The third sensor device SU-3 is installed in a living room RM-3 (not shown) of Person C Ob-3 who is one of the monitor subjects Ob. The fourth sensor device SU-4 is installed in a living room RM-4 (not shown) of Person D Ob-4 who is one of the monitor subjects Ob.

The management server device SV is a device having a communication function and the like to communicate with other devices SU, SP, and TA via the network NW. The device manages information (monitoring information) regarding monitoring of the monitor subject Ob upon receipt of a notification of the predetermined event related to the monitor subject Ob from the sensor device SU. Upon receipt of a first event notification communication signal from the sensor device SU as the notification of the event, based on each information item accommodated in the first event notification communication signal, the management server device SV stores (records) the monitoring information regarding monitoring of the monitor subject Ob. Then, the management server device SV transmits a communication signal (second event notification communication signal) accommodating the monitoring information regarding monitoring of the monitor subject Ob to the predetermined terminal devices SP and TA associated with the sensor device SU in advance. For this purpose, the management server device SV stores correspondence (notification destination correspondence) between a sensor ID that is a transmission source and a terminal ID that is a notification destination (re-notification destination), and the communication address. The correspondence indicates the notification destination (re-notification destination, re-report destination, transmission destination) of the first event notification communication signal and the like transmitted from the sensor device SU. The terminal ID (terminal device identifier) is an identifier for specifying and identifying the terminal devices SP and TA. Then, the management server device SV provides the client with data according to a request of the client (fixed terminal device SP, portable terminal device TA, or the like in the present embodiment). Such a management server device SV can be configured, for example, by a computer with a communication function.

The fixed terminal device SP is a device that functions as a user interface (UI) for the monitor-subject monitoring system MS. In order to implement this function, the fixed terminal device SP has functions such as a communication function of communicating with other devices SU, SV, and TA via the network NW, a display function of displaying predetermined information, and an input function of inputting predetermined instructions and data. The fixed terminal device SP receives the predetermined instructions and data to be given to the management server device SV and the portable terminal device TA, and displays the monitoring information obtained by the sensor device SU. Such a fixed terminal device SP can be configured, for example, by a computer with a communication function.

The portable terminal device TA is carried by the observer NS. The portable terminal device TA has functions such as a communication function of communicating with other devices SV, SP, and SU via the network NW, a display function of displaying predetermined information, an input function of inputting predetermined instructions and data, and a call function of performing a voice call. The portable terminal device TA is a device for inputting predetermined instructions and data to be given to the management server device SV and the sensor device SU, for displaying the monitoring information (including a moving image) obtained by the sensor device SU by the notification from the management server device SV, and for responding to a nurse call or calling by a voice call with the sensor device SU.

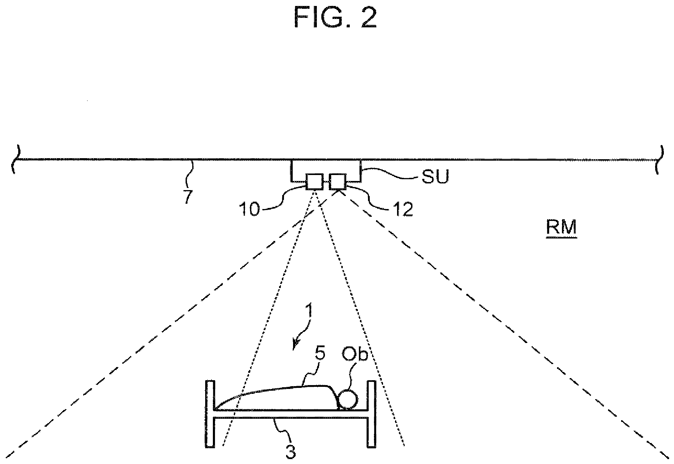

The sensor device SU will be described in detail. In addition to the functions described above (for example, the function of generating a moving image of the monitor subject Ob, the function of performing a voice call with the terminal devices SP and TA), the sensor device SU has a function of monitoring the living body of the monitor subject Ob by detecting a respiratory abnormality of the monitor subject Ob. The sensor device SU will be described in detail from a viewpoint of this function. FIG. 2 is a schematic view showing the living room RM in which the sensor device SU is installed. Bed space 1 for the monitor subject Ob is provided in the living room RM. The bed space 1 is a mattress 5 laid on a bed 3. The bed space 1 is not limited to the mattress 5 laid on the bed 3, and may be, for example, the mattress 5 laid on a tatami mat, or the mattress 5 laid on a floor. FIG. 2 shows the monitor subject Ob sleeping in the bed space 1. Only a head of the monitor subject Ob appears out of the body of the monitor subject Ob. The sensor device SU is attached to a ceiling 7 of the living room RM.

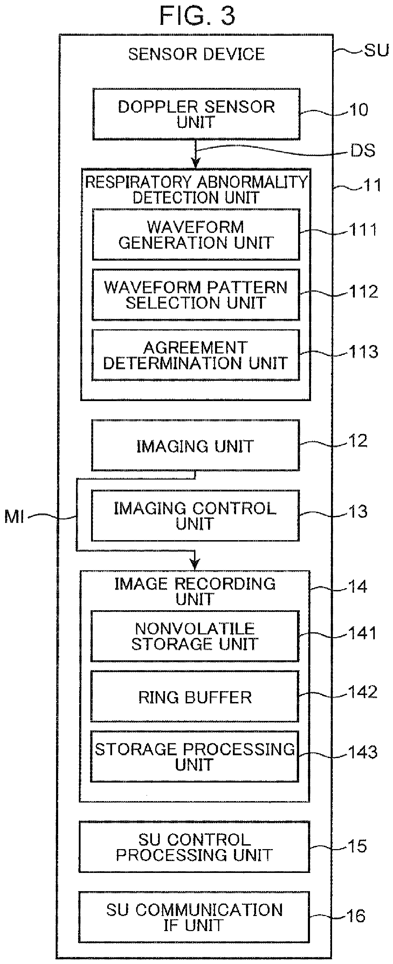

FIG. 3 is a block diagram showing a configuration of the sensor device SU included in the monitor-subject monitoring system MS according to the embodiment. The sensor device SU (living body monitoring device, respiratory abnormality detection device) includes a Doppler sensor unit 10, a respiratory abnormality detection unit 11, an imaging unit 12, an imaging control unit 13, an image recording unit 14, a sensor side control processing unit (SU control processing unit) 15, and a sensor side communication interface unit (SU communication IF unit) 16. In FIG. 2, among blocks that constitute the sensor device SU, the imaging unit 12 and the Doppler sensor unit 10 are shown, and other blocks are omitted.

The Doppler sensor unit 10 is a device for detecting a biological signal of the monitor subject Ob. The Doppler sensor unit 10 functions as an acquisition unit, and acquires the biological signal of the monitor subject Ob. The Doppler sensor unit 10 is a body motion sensor that transmits a transmission wave, receives a reflected wave of the transmission wave reflected by an object, and outputs a Doppler signal DS of a Doppler frequency component based on the transmission wave and the reflected wave. When the object is moving, a frequency of the reflected wave shifts in proportion to a speed at which the object is moving by the so-called Doppler effect, generating a difference between a frequency of the transmission wave and a frequency of the reflected wave (Doppler frequency component). The Doppler sensor unit 10 generates a signal of this Doppler frequency component as the Doppler signal DS. The transmission wave may be an ultrasonic wave, a microwave, or other waves, and is a microwave in the embodiment. The microwave is preferable because the microwave can penetrate clothes and be reflected by a body surface of the monitor subject Ob, enabling detection of movement of the body surface even if the monitor subject Ob wears clothes.

With reference to FIG. 2, the Doppler sensor unit 10 is installed on a ceiling surface above the bed 3 used by the monitor subject Ob. An installation location of the Doppler sensor unit 10 is not limited to this location. The Doppler sensor unit 10 may be installed, for example, under a mat of the bed 3 used by the monitor subject Ob, or may be installed at a position on a side of the bed 3 (position where the transmission wave is transmitted from a direction of the side of the bed 3 toward the monitor subject Ob).

With reference to FIG. 3, the respiratory abnormality detection unit 11 includes a waveform generation unit 111, a waveform pattern selection unit 112, and an agreement determination unit 113. The respiratory abnormality detection unit 11 functions as a detection unit. The detection unit detects whether respiration of the monitor subject Ob is abnormal based on the biological signal acquired by the acquisition unit (Doppler sensor unit 10).

The waveform generation unit 111 extracts a respiratory signal component from the Doppler signal DS output from the Doppler sensor unit 10 by using wavelet analysis or the like. By using this respiratory signal component, the waveform generation unit 111 generates the respiratory signal indicating a relationship between time and signal strength. A waveform of this respiratory signal shows a respiratory state of the monitor subject Ob. In this way, the waveform generation unit 111 functions as a generation unit, and generates the respiratory signal indicating the respiratory state of the monitor subject Ob by using the biological signal acquired by the acquisition unit (Doppler sensor unit 10).

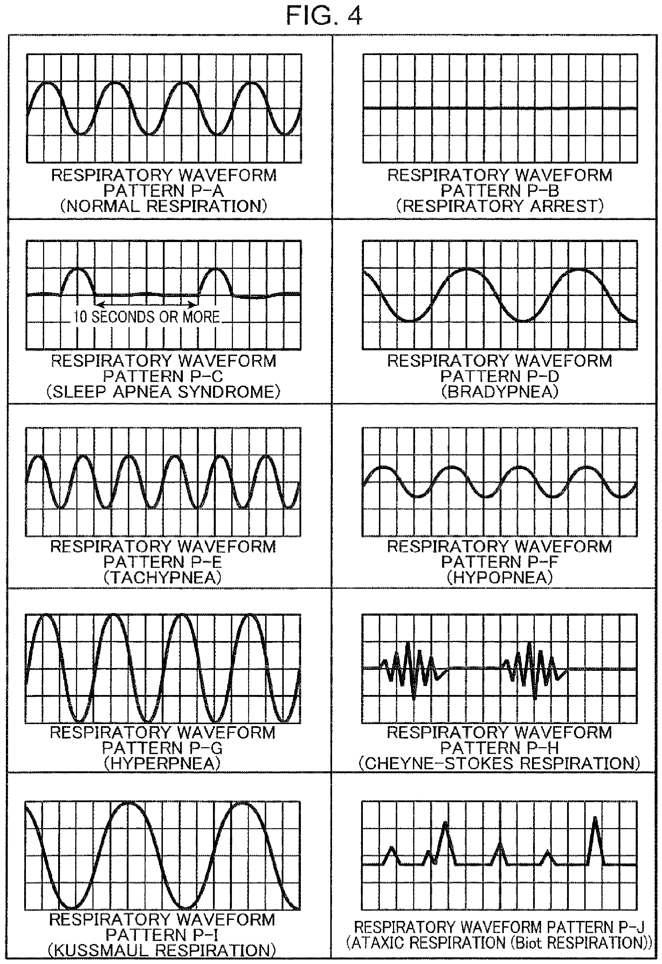

Respiratory waveform patterns indicated by the respiratory signal will be described. FIG. 4 is an explanatory diagram describing various types of respiratory waveform patterns P. FIG. 4 shows a respiratory waveform pattern P-A of normal respiration, a respiratory waveform pattern P-B of respiratory arrest, a respiratory waveform pattern P-C of sleep apnea syndrome, a respiratory waveform pattern P-D of bradypnea, a respiratory waveform pattern P-E of tachypnea, a respiratory waveform pattern P-F of hypopnea, a respiratory waveform pattern P-G of hyperpnea, a respiratory waveform pattern P-H of Cheyne-Stokes respiration, a respiratory waveform pattern P-I of Kussmaul respiration, and a respiratory waveform pattern P-J of ataxic respiration (Biot respiration).

The respiratory waveform patterns P other than the respiratory waveform pattern P-A of normal respiration are respiratory waveform patterns P of respiratory abnormalities. The respiratory waveform patterns P of abnormal respiration differ from one another in waveform patterns. In the respiratory waveform pattern P-B of respiratory arrest, the respiratory waveform is continuously flat. When the respiratory waveform is continuously flat, the respiratory abnormality detection unit 11 determines respiratory arrest. It is determined using pattern matching to be described later whether the respiratory waveform pattern corresponds to respiratory abnormalities other than respiratory arrest. The respiratory waveform patterns P of respiratory abnormalities to undergo pattern matching are eight types (respiratory waveform pattern P-C to respiratory waveform pattern P-J). These respiratory waveform patterns P are specific examples of the plurality of respiratory waveform patterns indicating the plurality of respiratory abnormalities.

As the number of types of respiratory waveform patterns of respiratory abnormalities to undergo pattern matching increases, a possibility of erroneous determination increases in pattern matching for a reason that there are similar patterns in these respiratory waveform patterns or other reasons. The erroneous determination includes determining normal respiration as a respiratory abnormality, and determining the type of respiratory abnormality by mistake. The former causes the observer NS to run unnecessarily, whereas the latter causes a delay in addressing the respiratory abnormality. Therefore, the embodiment lowers a possibility of performing erroneous determination in pattern matching by reducing the number of types of respiratory waveform patterns of respiratory abnormalities to undergo pattern matching based on an indicator indicating a health condition of the monitor subject Ob.

It is known that there is a relationship between the indicator indicating the health condition of the monitor subject Ob and the type of respiratory abnormality. An example will be described in which the indicator indicating the health condition is body weight. It is known that when body weight decreases significantly, a possibility of one of bradypnea, Cheyne-Stokes respiration, and ataxic respiration (Biot respiration) is high, and that a possibility of other types of respiratory abnormality is low. The present inventor has created one example of a table indicating a relationship between the indicator indicating the health condition of the monitor subject Ob and the type of respiratory abnormality. Table 1 shows this table. Examples of the indicator indicating the health condition are body temperature, body weight, blood pressure, dietary intake, and sleeping hours.

**[Table 1]**

| | Condition | | P-C | P-D | P-E | P-F | P-G | P-H | P-I | P-J |
|---|---|---|---|---|---|---|---|---|---|---|
| Body temperature | C-1 | 37.5°C or higher | | | ○ | | | | | |
| | C-2 | 35.5°C or lower | | | ○ | | ○ | | | |
| Body weight | C-3 | Decrease by 10% or more | | ○ | | | | ○ | | ○ |
| | C-4 | Increase by 10% or more | ○ | | | ○ | | | ○ | |
| Blood pressure | C-5 | Decrease by 10% or more in minimal blood pressure | | ○ | ○ | | | ○ | | ○ |
| | C-6 | Increase by 10% or more in maximal blood pressure | ○ | | | ○ | | | ○ | |
| Dietary intake | C-7 | Decrease by 20% or more | | ○ | ○ | | | ○ | | ○ |
| Sleeping hours | C-8 | Decrease by 20% or more | ○ | | | ○ | ○ | | | |

When the body temperature in the daytime is high (for example, 37.5 degrees or higher), the respiratory waveform pattern P of respiratory abnormality to undergo pattern matching is the respiratory waveform pattern P-E (tachypnea). When the body temperature in the daytime is low (for example, 35.5 degrees or lower), the respiratory waveform pattern P of respiratory abnormality to undergo pattern matching is the respiratory waveform pattern P-E (tachypnea) and the respiratory waveform pattern P-G (hyperpnea). The body temperature in the daytime is a body temperature measured when the monitor subject Ob is awake other than when the monitor subject Ob is sleeping. Generally, in a welfare facility for the aged or the like, the body temperature of the monitor subject Ob is measured after breakfast, before bathing (around 15:00), and after supper.

When the body weight is decreasing (for example, when the body weight is becoming lighter than one month before by 10% or more), the respiratory waveform pattern P of respiratory abnormality to undergo pattern matching is the respiratory waveform pattern P-D (bradypnea), the respiratory waveform pattern P-H (Cheyne-Stokes respiration), and the respiratory waveform pattern P-J (ataxic respiration (Biot respiration)). When the body weight is increasing (for example, when the body weight is becoming heavier than one month before by 10% or more), the respiratory waveform pattern P of respiratory abnormality to undergo pattern matching is the respiratory waveform pattern P-C (sleep apnea syndrome), the respiratory waveform pattern P-F (hypopnea), and the respiratory waveform pattern P-I (Kussmaul respiration).

When the blood pressure is decreasing (for example, when a diastolic blood pressure of ordinary times (minimal blood pressure) is becoming lower than one week before by 10% or more), the respiratory waveform pattern P of respiratory abnormality to undergo pattern matching is the respiratory waveform pattern P-D (bradypnea), the respiratory waveform pattern P-E (tachypnea), the respiratory waveform pattern P-H (Cheyne-Stokes respiration), and the respiratory waveform pattern P-J (ataxic respiration (Biot respiration)). When the blood pressure is increasing (for example, when a systolic blood pressure of ordinary times (maximal blood pressure) is becoming higher than one week before by 10% or more), the respiratory waveform pattern P of respiratory abnormality to undergo pattern matching is the respiratory waveform pattern P-C (sleep apnea syndrome), the respiratory waveform pattern P-F (hypopnea), and the respiratory waveform pattern P-I (Kussmaul respiration).

When the dietary intake is decreasing (for example, when the dietary intake is becoming smaller than three days before by 20% or more), the respiratory waveform pattern P of respiratory abnormality to undergo pattern matching is the respiratory waveform pattern P-D (bradypnea), the respiratory waveform pattern P-E (tachypnea), the respiratory waveform pattern P-H (Cheyne-Stokes respiration), and the respiratory waveform pattern P-J (ataxic respiration (Biot respiration)).

When the sleeping hours are decreasing (for example, when the sleeping hours are becoming shorter than one week before by 20% or more), the respiratory waveform pattern P of respiratory abnormality to undergo pattern matching is the respiratory waveform pattern P-C (sleep apnea syndrome), the respiratory waveform pattern P-F (hypopnea), and the respiratory waveform pattern P-G (hyperpnea).

As described above, the number of types of respiratory waveform patterns P to undergo pattern matching can be reduced based on the indicator indicating the health condition of the monitor subject Ob.

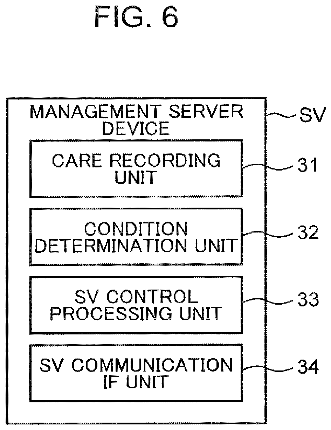

With reference to FIG. 3, based on a result determined by a condition determination unit 32 to be described later (FIG. 6), the waveform pattern selection unit 112 selects one or more respiratory waveform patterns P to undergo pattern matching from the respiratory waveform pattern P-C to the respiratory waveform pattern P-J. The respiratory waveform pattern P-C to the respiratory waveform pattern P-J are specific examples of the plurality of waveform patterns indicating the plurality of types of respiratory abnormalities. With reference to FIG. 4 and Table 1, for example, when the body temperature of the monitor subject Ob is 35.5 degrees or lower, the waveform pattern selection unit 112 selects the respiratory waveform pattern P-E (tachypnea) and the respiratory waveform pattern P-G (hyperpnea). This is because, when the body temperature of the monitor subject Ob is 35.5 degrees or lower, there is a possibility of tachypnea or hyperpnea, but there is no (almost no) possibility of respiratory abnormalities other than tachypnea or hyperpnea. Thus, the waveform pattern selection unit 112 functions as a selection unit, and selects one or more respiratory waveform patterns P from the plurality of respiratory waveform patterns P indicating the plurality of types of respiratory abnormalities by using the predetermined indicator serving as the indicator indicating the health condition of the monitor subject Ob.

The agreement determination unit 113 performs pattern matching between the waveform of the respiratory signal generated by the waveform generation unit 111 and each of one or more respiratory waveform patterns P selected by the waveform pattern selection unit 112. Accordingly, the agreement determination unit 113 determines whether the waveform of the respiratory signal generated by the waveform generation unit 111 agrees with one of the one or more respiratory waveform patterns P selected by the waveform pattern selection unit 112. Thus, the agreement determination unit 113 functions as a determination unit, and determines whether the waveform of the respiratory signal generated by the generation unit (waveform generation unit 111) corresponds to one of the one or more respiratory waveform patterns P selected by the selection unit (waveform pattern selection unit 112), by using the waveform of the respiratory signal generated by the generation unit and the one or more respiratory waveform patterns P selected by the selection unit.

When the agreement determination unit 113 determines agreement, the agreement determination unit 113 determines that the respiratory abnormality associated with the corresponding respiratory waveform pattern P is the type of respiratory abnormality of the monitor subject Ob. Note that a condition that the respiratory rate for 1 minute is 9 or less is further added to determination of bradypnea (FIG. 4). A condition that the respiratory rate for 1 minute is 25 or more is further added to determination of tachypnea (FIG. 4). A condition that amplitude of the respiratory waveform continuously decreases to 50% or less of amplitude of the respiratory waveform of ordinary times for 10 seconds or more is further added to determination of hypopnea (FIG. 4).

As described above, in addition to the pattern matching, the respiratory abnormality detection unit 11 measures the respiratory rate for 1 minute based on the respiratory signal, and measures an amplitude value of the respiratory waveform indicated by the respiratory signal. By using these measurements, the respiratory abnormality detection unit 11 detects whether respiration is abnormal, and the type of respiratory abnormality.

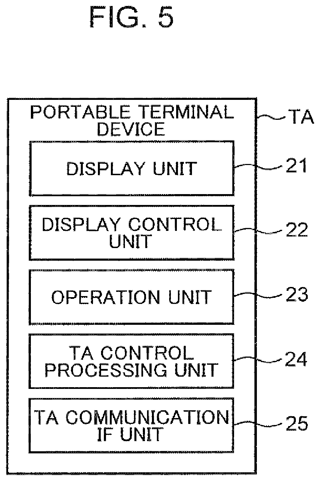

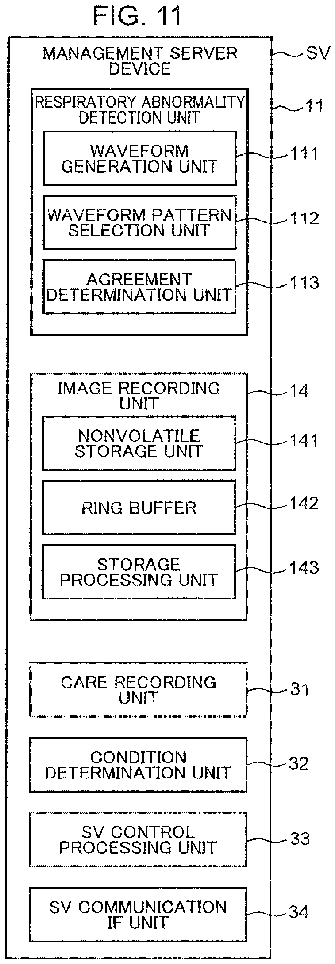

The respiratory abnormality detection unit 11 is implemented by hardware such as, for example, a central processing unit (CPU), a random access memory (RAM), a read only memory (ROM), and a hard disk drive (HDD), and a program and data for performing functions of the respiratory abnormality detection unit 11. In place of or in addition to processing by the CPU, part or all of the functions of the respiratory abnormality detection unit 11 may be implemented by processing by a digital signal processor (DSP). Similarly, in place of or in addition to processing by software, part or all of the functions of the respiratory abnormality detection unit 11 may be implemented by processing by a dedicated hardware circuit. The above description is also applied to the following units to be described later: the imaging control unit 13 (FIG. 3, FIG. 8, FIG. 10), the image recording unit 14 (FIG. 3, FIG. 8, FIG. 11), the SU control processing unit 15 (FIG. 3, FIG. 8, FIG. 10), a display control unit 22 (FIG. 5), a TA control processing unit 24 (FIG. 5), a care recording unit 31 (FIG. 6, FIG. 11), the condition determination unit 32 (FIG. 6, FIG. 11), and an SV control processing unit 33 (FIG. 6, FIG. 11).

The imaging unit 12 is a device for capturing an image of an imaging target and generating an image of the imaging target. The image includes a still image and a moving image. The imaging unit 12 is disposed to enable monitoring of a space where the monitor subject Ob is to be located (location space, the living room RM of the installation place in the example shown in FIG. 1). The imaging unit 12 captures an image of the location space from above as the imaging target, and generates an image that overlooks the imaging target. Due to high probability of being able to capture the entire monitor subject Ob, the imaging unit 12 is preferably installed such that an image of the imaging target can be captured from right above an planned head position (typically, installment position of a pillow) set in advance at which a head of the monitor subject Ob is expected to be positioned in bedding (for example, bed) on which the monitor subject Ob lies. With the imaging unit 12, the sensor device SU acquires an image obtained by capturing the monitor subject Ob from above the monitor subject Ob, preferably, an image obtained by capturing the monitor subject Ob from right above the planned head position.

Such an imaging unit 12 may be a device that generates an image of visible light, but to allow monitoring of the monitor subject Ob in a relatively dimly lighted place, in the embodiment, the imaging unit 12 is a device that generates an infrared image. In the embodiment, such an imaging unit 12 is, for example, a digital infrared camera including components such as: an image formation optical system, an image sensor, and an image processing unit. The image formation optical system forms an infrared optical image of the imaging target on a predetermined image formation surface. The image sensor is disposed with a light-receiving surface aligned with the image formation surface, and converts the infrared optical image of the imaging target into an electric signal. The image processing unit generates image data that is data representing the infrared image of the imaging target by performing image processing on an output of the image sensor. In the embodiment, the image formation optical system of the imaging unit 12 is preferably a wide-angle optical system (so-called wide-angle lens (including a fish-eye lens)) having an angle of view that can capture the entire living room RM where the image formation optical system is installed.

The imaging control unit 13 controls the imaging unit 12, and causes the imaging unit 12 to capture a moving image of the imaging target. Instead of the moving image, a plurality of time-series still images (slide images) may be captured. Thus, the imaging control unit 13 functions as a control unit, and has a function of causing the imaging unit 12 to capture the monitor subject Ob continuously.

The image recording unit 14 records an image of the monitor subject Ob. The image recording unit 14 includes a nonvolatile storage unit 141, a ring buffer 142, and a storage processing unit 143.

The nonvolatile storage unit 141 (storage unit) has a function of holding stored information (data) even if the sensor device SU is turned off (that is, nonvolatile), and is implemented by a hard disk drive, a flash memory, or the like.

The ring buffer 142 can store information (data) of a predetermined quantity, and has a function of, when storing new information (data), storing information of a predetermined quantity (data) endlessly by deleting data in order from old information.

The storage processing unit 143 causes the ring buffer 142 to store the moving image captured by the imaging unit 12. This allows the ring buffer 142 to endlessly store the moving image including frames before predetermined time from the latest frame.

When a respiratory abnormality is detected by the respiratory abnormality detection unit 11, the storage processing unit 143 reads, from the ring buffer 142, a frame captured at predetermined timing before the respiratory abnormality is detected, and stores the frame in the nonvolatile storage unit 141 (storage unit). This frame indicates an image of the monitor subject Ob before the respiratory abnormality is detected. This frame indicates a posture in which the monitor Ob is sleeping and a direction of a face or the like, before the respiratory abnormality is detected. Therefore, this frame serves as information for investigating a cause of the respiratory abnormality.

The frame captured at predetermined timing is, for example, a frame captured several seconds to several tens of seconds before reference timing when the respiratory abnormality is detected (for example, a frame captured five seconds before). Accordingly, a still image (one frame) of the monitor subject Ob immediately before the respiratory abnormality is detected is stored in the nonvolatile storage unit 141. Not only a still image but a moving image may be stored.

Note that the storage processing unit 143 may generate a silhouette image obtained by making a silhouette of the image of the monitor subject Ob included in the frame (image) read from the ring buffer 142, and store the silhouette image in the nonvolatile storage unit 141. The silhouette is an image obtained by filling an inside of an outline with single color. The silhouette of the monitor subject Ob does not indicate the face of the monitor subject Ob. Therefore, even if a situation occurs where the image stored in the nonvolatile storage unit 141 flows out, privacy of the monitor subject Ob can be protected. Since the silhouette image indicates the posture in which the monitor subject Ob is sleeping and the direction of the face or the like, the silhouette image serves as information for investigating a cause of a respiratory abnormality.

As described above, the storage processing unit 143 functions as a processing unit. When the respiratory abnormality of the monitor subject Ob is detected by the detection unit (respiratory abnormality detection unit 11), the processing unit acquires the image of the monitor subject Ob captured by the imaging unit 12 during a time period including the time when the respiratory abnormality is detected, and causes the nonvolatile storage unit 141 to store the acquired image.

The SU communication IF unit 16 is a communication circuit connected to the SU control processing unit 15 for performing communication according to control of the SU control processing unit 15. The SU communication IF unit 16 generates a communication signal accommodating data that is to be transferred and input from the SU control processing unit 15 in compliance with a communication protocol used in the network NW of the monitor-subject monitoring system MS. The SU communication IF unit 16 transmits this generated communication signal to other devices SV, SP, and TA via the network NW. The SU communication IF unit 16 receives the communication signal from other devices SV, SP, and TA via the network NW, and extracts the data from this received communication signal. The SU communication IF unit 16 converts this extracted data into data of a format that can be processed by the SU control processing unit 15, and outputs the converted data to the SU control processing unit 15. The SU communication IF unit 16 includes, for example, a communication interface circuit in compliance with the IEEE 802.11 standard and other standards.

The SU control processing unit 15 is a device for controlling each unit of the sensor device SU (Doppler sensor unit 10, respiratory abnormality detection unit 11, imaging unit 12, imaging control unit 13, image recording unit 14, SU communication IF unit 16) according to a function of the unit, for detecting a predetermined event related to the monitor subject Ob and notifying the management server device SV of this detected event, for performing a voice call with the terminal devices SP and TA, and for distributing a moving image and an image including a moving image to the terminal devices SP and TA.

The sensor device SU functions as a living body monitoring device by including the respiratory abnormality detection unit 11 and the image recording unit 14. From this viewpoint, the monitor-subject monitoring system MS serves as a living body monitoring system.

The sensor device SU functions as a respiratory abnormality detection device by including the respiratory abnormality detection unit 11. From this viewpoint, the monitor-subject monitoring system MS serves as a respiratory abnormality detection system. In this case, the sensor device SU does not need to include the imaging unit 12, the imaging control unit 13, and the image recording unit 14.

The portable terminal device TA will be described in detail. FIG. 5 is a block diagram showing a configuration of the portable terminal device TA included in the monitor-subject monitoring system MS according to the embodiment. The portable terminal device TA includes a display unit 21, a display control unit 22, an operation unit 23, a terminal side control processing unit (TA control processing unit) 24, and a terminal side communication interface unit (TA communication IF unit) 25.

The display unit 21 is implemented by a liquid crystal display or the like. The display control unit 22 performs control to cause the display unit 21 to display an image (for example, a moving image). The operation unit 23 is a device for inputting an instruction or the like for operating the portable terminal device TA. The operation unit 23 is implemented by a touch panel or the like.

The TA communication IF unit 25 is a communication circuit connected to the TA control processing unit 24 for performing communication according to control of the TA control processing unit 24. The TA communication IF unit 25 generates a communication signal accommodating data that is to be transferred and input from the TA control processing unit 24 in compliance with a communication protocol used in the network NW of the monitor-subject monitoring system MS. The TA communication IF unit 25 transmits this generated communication signal to other devices SU, SV, and SP via the network NW. The TA communication IF unit 25 receives the communication signal from other devices SU, SV, and SP via the network NW, and extracts the data from this received communication signal. The TA communication IF unit 25 converts this extracted data into data of a format that can be processed by the TA control processing unit 24, and outputs the converted data to the TA control processing unit 24. The TA communication IF unit 25 includes, for example, a communication interface circuit in compliance with the IEEE 802.11 standard and other standards.

The TA control processing unit 24 is a device for controlling each unit of the portable terminal device TA (display unit 21, display control unit 22, operation unit 23, TA communication IF unit 25) according to a function of the unit, for receiving and displaying monitoring information of the monitor subject Ob, for receiving input of implementation intention information, and for responding to a nurse call or calling.

The observer NS operates the operation unit 23 of the portable terminal device TA and inputs information serving as a care record of the monitor subject Ob every day. The information includes vital data (for example, body temperature, body weight, maximal blood pressure, minimal blood pressure, and the like), daily dietary intake, and daily sleeping hours. The TA control processing unit 24 orders the TA communication IF unit 25 to transmit the information to the management server device SV. The TA communication IF unit 25 transmits the information to the management server SV The information is recorded in the care recording unit 31 to be described later (FIG. 6) as the care record.

The management server device SV will be described. FIG. 6 is a block diagram showing a configuration of the management server device SV included in the monitor-subject monitoring system MS according to the embodiment. The management server device SV includes the care recording unit 31, the condition determination unit 32, the management server side control processing unit (SV control processing unit) 33, and a management server side communication interface unit (SV communication IF unit) 34.

The care recording unit 31 creates and stores the care record for each monitor subject Ob. The care record is created in one day, and includes vital data (for example, body temperature, body weight, maximal blood pressure, minimal blood pressure, and the like), daily dietary intake, and daily sleeping hours.

The condition determination unit 32 refers to the care record of the monitor subject Ob at timing determined in advance, and determines whether the health condition of the monitor subject Ob corresponds to one of eight conditions C shown in Table 1 (condition C-1 to condition C-8) for each monitor subject Ob. The timing determined in advance is, for example, timing when body temperature information indicating the body temperature measured after supper (body temperature measured at the end of one day) is sent from the portable terminal device TA to the care recording unit 31, and the care recording unit 31 records the body temperature indicated by the body temperature information in the care record.

The SV communication IF unit 34 is a communication circuit connected to the SV control processing unit 33 for performing communication according to control of the SV control processing unit 33. The SV communication IF unit 34 is a communication circuit similar to the SU communication IF unit 16, and includes, for example, a communication interface circuit in compliance with the IEEE 802.11 standard and other standards.

The SV control processing unit 33 is a device for controlling each unit of the management server device SV (care recording unit 31, condition determination unit 32, SV communication IF unit 34) according to a function of the unit, for managing the monitoring information regarding monitoring of the monitor subject Ob upon receipt of notification of the predetermined event from the sensor device SU, for notifying (transmitting) a client (here, predetermined terminal devices SP and TA) of the predetermined event, for providing the client with data in response to a request of the client, and for managing the overall monitor-subject monitoring system MS.

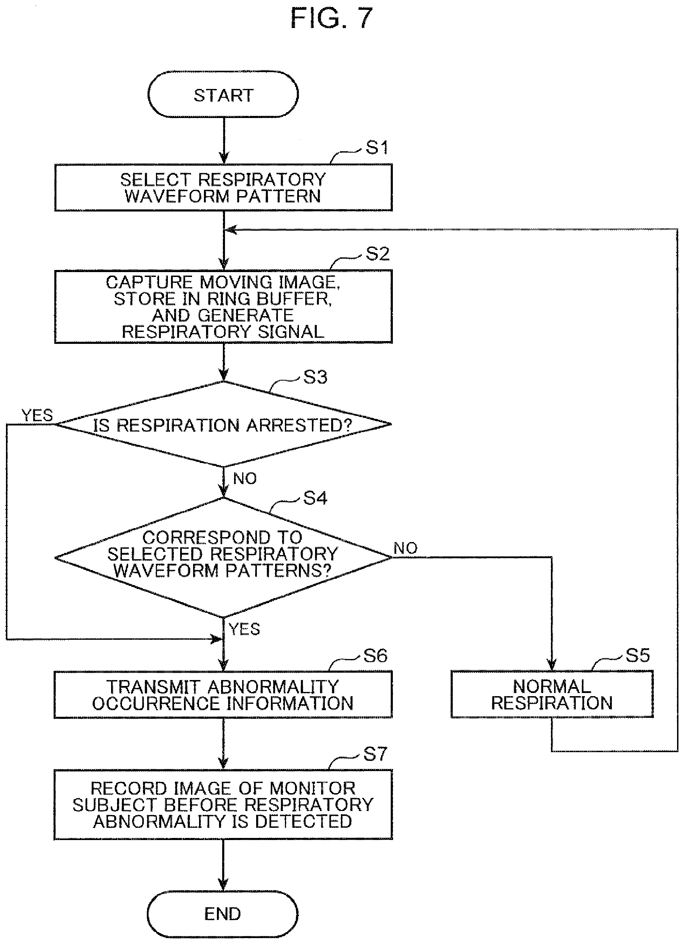

Respiratory abnormality detection and image recording to be performed by the monitor-subject monitoring system MS according to the embodiment will be described. FIG. 7 is a flowchart describing the respiratory abnormality detection and the image recording. The monitor subject Ob-1 (FIG. 1) is taken as an example. With reference to FIG. 6, by using the care record of the monitor subject Ob-1 recorded in the care recording unit 31, the condition determination unit 32 determines whether the care record corresponds to one of the eight conditions C shown in Table 1. The care record may correspond to none of the eight conditions C, may correspond to one of the eight conditions C, or may correspond to two or more of the eight conditions C. Here, an example will be described in which the care record corresponds to the condition C-4 (when the body weight is becoming heavier by 10% or more compared to one month before). The SV control processing unit 33 orders the SV communication IF unit 34 to transmit the condition information indicating the condition C-4 to the sensor device SU-1. The SV communication IF unit 34 transmits the condition information to the sensor device SU-1.

With reference to FIG. 3, the SU communication IF unit 16 of the sensor device SU-1 receives the condition information, and the SU control processing unit 15 causes the waveform pattern selection unit 112 to store the condition information. The waveform pattern selection unit 112 selects the respiratory waveform pattern P to undergo pattern matching from the respiratory waveform pattern P-C to the respiratory waveform pattern P-J (FIG. 4) stored in advance based on the condition shown by the condition information (step S1). Here, since the care record corresponds to the condition C-4, the respiratory waveform pattern P-C (sleep apnea syndrome), the respiratory waveform pattern P-F (hypopnea), and the respiratory waveform pattern P-I (Kussmaul respiration) are selected.

In the sensor device SU-1, the imaging unit 12 captures a moving image of an imaging target including the monitor subject Ob-1, and the storage processing unit 143 stores the captured moving image in the ring buffer 142 (step S2). In parallel to this operation, the waveform generation unit 111 extracts the respiratory signal component from the Doppler signal DS output from the Doppler sensor unit 10. By using this respiratory signal component, the waveform generation unit 111 generates a respiratory signal indicating a relationship between time and signal strength (step S2). The waveform of the respiratory signal indicates a respiratory state of the monitor subject Ob-1.

By using the respiratory signal generated in step S2, the respiratory abnormality detection unit 11 determines whether respiration is arrested (step S3). More particularly, when the respiratory waveform is continuously flat for a period determined in advance, the respiratory abnormality detection unit 11 determines that respiration is arrested.

When the respiratory abnormality detection unit 11 determines that respiration is not arrested (No in step S3), the agreement determination unit 113 determines whether the waveform of the respiratory signal generated in step S2 corresponds to one of the respiratory waveform pattern P-C (sleep apnea syndrome), the respiratory waveform pattern P-F (hypopnea), and the respiratory waveform pattern P-I (Kussmaul respiration) by using pattern matching (step S4). Note that when the waveform of the respiratory signal generated in step S2 corresponds to none of the eight conditions C shown in Table 1, the process of step S4 is not performed.

When the agreement determination unit 113 determines that the waveform of the respiratory signal generated in step S2 corresponds to none of the respiratory waveform pattern P-C (sleep apnea syndrome), the respiratory waveform pattern P-F (hypopnea), and the respiratory waveform pattern P-I (Kussmaul respiration) (No in step S4), the respiratory abnormality detection unit 11 determines that the respiratory state of the monitor subject Ob-1 is normal respiration (step S5), and returns to the process of step S2.

When the agreement determination unit 113 determines that the respiratory signal generated in step S2 corresponds to one of the respiratory waveform pattern P-C (sleep apnea syndrome), the respiratory waveform pattern P-F (hypopnea), and the respiratory waveform pattern P-I (Kussmaul respiration) (Yes in step S4), the SU control processing unit 15 orders the SU communication IF unit 16 to transmit abnormality occurrence information to the management server device SV. The abnormality occurrence information includes occurrence of a respiratory abnormality, information for identifying the monitor subject Ob-1 in which the respiratory abnormality has occurred (for example, name of the monitor subject Ob-1 and a number of the living room RM), and a type of respiratory abnormality. The abnormality occurrence information further includes an image of the monitor subject Ob-1 stored in the nonvolatile storage unit 141 in step S7 to be described later. The SU communication IF unit 16 transmits the abnormality occurrence information to the management server device SV (step S6).

With reference to FIG. 5 and FIG. 6, the management server device SV receives the abnormality occurrence information, and transfers the received abnormality occurrence information to each portable terminal device TA. Accordingly, each portable terminal device TA informs the observer NS of the abnormality occurrence. For example, the portable terminal device TA sounds an alarm sound and causes the display unit 21 to display an image indicating the abnormality occurrence. This image includes the image of the monitor subject Ob-1 stored in the nonvolatile storage unit 141 in step S7. Note that when the storage processing unit 143 shown in FIG. 3 performs processing for generating the silhouette image (image obtained by converting the image of the monitor subject Ob-1 into a silhouette) from the image of the monitor subject Ob-1 and causes the nonvolatile storage unit 141 to store the silhouette image, the image of the monitor subject Ob-1 included in the abnormality occurrence information is not the silhouette image but the image of the monitor subject Ob-1 before the silhouette processing is performed (image with which the face of the monitor subject Ob-1 can be recognized). When switching the image to be displayed on the display unit 21 from the image of the monitor subject Ob-1 before the silhouette processing to another image, the display control unit 22 deletes data of the image of the monitor subject Ob-1 before the silhouette processing. Accordingly, since the image of the monitor subject Ob-1 before the silhouette processing is not stored in the memory of the portable terminal device TA, privacy of the monitor subject Ob-1 can be protected.

With reference to FIG. 3 and FIG. 7, in parallel to step S6, the storage processing unit 143 reads, from the ring buffer 142, a frame captured at predetermined timing before the respiratory abnormality is detected by the respiratory abnormality detection unit 11 (for example, a frame captured five seconds before), and causes the nonvolatile storage unit 141 to store the frame. Accordingly, the image of the monitor subject Ob-1 before the respiratory abnormality is detected is recorded (step S7).

When the respiratory abnormality detection unit 11 determines that respiration of the monitor subject Ob-1 is arrested (Yes in step S3), the respiratory abnormality detection unit 11 performs the process of step S6 and step S7. The abnormality occurrence information of step S6 includes occurrence of respiratory arrest, and information for identifying the monitor subject Ob-1 in which the respiratory arrest has occurred (for example, the name of the monitor subject Ob-1 and the number of the living room RM). The storage processing unit 143 reads, from the ring buffer 142, a frame captured at predetermined timing before the respiratory arrest is determined by the respiratory abnormality detection unit 11 (for example, a frame captured five seconds before), and causes the nonvolatile storage unit 141 to store the frame. Accordingly, the image of the monitor subject Ob-1 before the respiratory arrest is determined is recorded (step S7).

Major effects of the embodiment include effects 1 to 4. Effect 1 will be described first. With reference to FIG. 3 and FIG. 7, when a respiratory abnormality of the monitor subject Ob is detected (Yes in step S3, Yes in step S4), the sensor device SU (living body monitoring device, respiratory abnormality detection device) reads, from the ring buffer 142, the frame (image) of the monitor subject Ob captured at predetermined timing before the respiratory abnormality is detected, and causes the nonvolatile storage unit 141 to store the frame (step S7). In this frame, the posture in which the monitor subject Ob is sleeping, and the direction of the face or the like appear. Therefore, according to the embodiment, when the respiratory abnormality of the monitor subject Ob is detected, the posture in which the monitor subject Ob is sleeping, and information regarding the direction of the face or the like can be acquired simultaneously.

Effect 2 will be described. In the frame captured at predetermined timing before a respiratory abnormality of the monitor subject Ob is detected (for example, the frame captured several seconds to several tens of seconds before the respiratory abnormality is detected), the posture in which the monitor subject Ob is sleeping, and the direction of the face or the like immediately before the respiratory abnormality of the monitor subject Ob is detected appear. The posture and the direction serve as information important for investigating a cause of the respiratory abnormality. With reference to FIG. 3 and FIG. 7, before the respiratory abnormality of the monitor subject Ob is detected, the embodiment causes the imaging unit 12 to capture a moving image MI of the monitor subject Ob, and stores the moving image MI in the ring buffer 142 (step S2). This makes it possible to cause the nonvolatile storage unit 141 to store the frame captured at predetermined timing before the respiratory abnormality of the monitor subject Ob is detected, without a large-capacity storage device (step S7).

Effect 3 will be described. With reference to FIG. 3 and FIG. 7, by using the predetermined indicator serving as the indicator indicating the health condition of the monitor subject Ob, the embodiment selects one or more respiratory waveform patterns from among the plurality of respiratory waveform patterns indicating the plurality of types of respiratory abnormalities (step S1). Since this makes it possible to reduce the number of types of respiratory waveform patterns of respiratory abnormalities (since the types can be narrowed down), erroneous determination can be reduced in the determination of whether the waveform of the respiratory signal of the monitor subject Ob corresponds to the respiratory waveform patterns of respiratory abnormalities (step S4). Therefore, according to the present embodiment, determination accuracy of whether respiration is abnormal, and determination accuracy of the type of respiratory abnormality can be improved.

Effect 4 will be described. With reference to FIG. 4 and FIG. 7, in the determination of whether the waveform of the respiratory signal of the monitor subject Ob corresponds to the respiratory waveform patterns P of respiratory abnormalities (step S4), combinations that are easy to cause erroneous determination on the grounds that the waveform patterns are similar are known experientially. These combinations include a combination of the respiratory waveform pattern P-C (sleep apnea syndrome) and the respiratory waveform pattern P-D (bradypnea), and a combination of the respiratory waveform pattern P-D (bradypnea) and the respiratory waveform pattern P-I (Kussmaul respiration).

When the monitor subject Ob has a sleep apnea syndrome, the agreement determination unit 113 (FIG. 3) should determine that the waveform of the respiratory signal of the monitor subject Ob agrees with the respiratory waveform pattern P-C, but may determine that the waveform of the respiratory signal of the monitor subject Ob agrees with the respiratory waveform pattern P-D. Conversely, when the monitor subject Ob has bradypnea, the agreement determination unit 113 should determine that the waveform of the respiratory signal of the monitor subject Ob agrees with the respiratory waveform pattern P-D, but may determine that the waveform of the respiratory signal of the monitor subject Ob agrees with the respiratory waveform pattern P-C. This also applies to a combination of the respiratory waveform pattern P-D and the respiratory waveform pattern P-I.

In the embodiment, since the waveform pattern selection unit 112 selects the respiratory waveform pattern P by using the conditions C shown in Table 1 (step S1), the waveform pattern selection unit 112 may not simultaneously select the respiratory waveform pattern P-C and the respiratory waveform pattern P-D. For example, when the waveform of the respiratory signal corresponds only to the condition C-4 shown in Table 1, the respiratory waveform pattern P-C is selected, but the respiratory waveform pattern P-D is not selected. In such a case, the agreement determination unit 113 should determine that the waveform of the respiratory signal of the monitor subject Ob agrees with the respiratory waveform pattern P-C, but determination that the waveform of the respiratory signal of the monitor subject Ob agrees with the respiratory waveform pattern P-D does not occur. This also applies to a combination of the respiratory waveform pattern P-D and the respiratory waveform pattern P-I.

There are modification 1 and modification 2 for the embodiment. Modification 1 will be described first. The embodiment causes the nonvolatile storage unit 141 to store the image of the monitor subject Ob before a respiratory abnormality is detected. Meanwhile, modification 1 causes the nonvolatile storage unit 141 to store an image of the monitor subject Ob when a respiratory abnormality is detected.

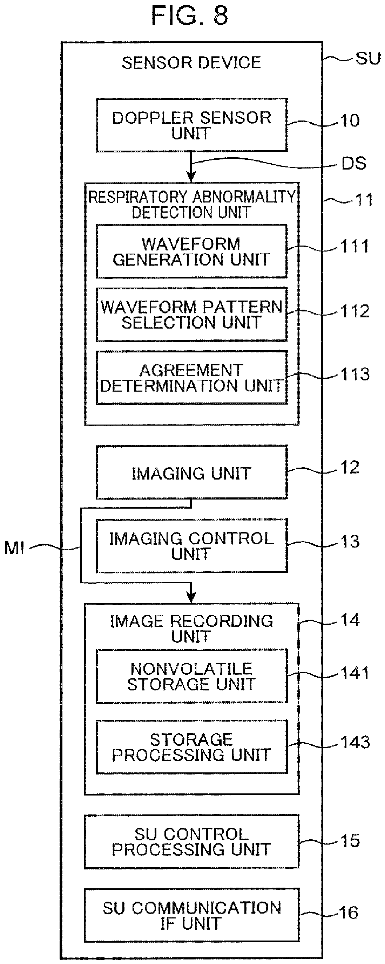

FIG. 8 is a block diagram showing a configuration of a sensor device SU included in modification 1. A difference from the sensor device SU included in the embodiment shown in FIG. 3 will be described. Modification 1 differs from the embodiment in a configuration of the image recording unit 14. In modification 1, when a respiratory abnormality detection unit 11 detects a respiratory abnormality, an imaging control unit 13 causes an imaging unit 12 to capture an image of an imaging target including the monitor subject Ob-1. A storage processing unit 143 causes a nonvolatile storage unit 141 to store the image. According to modification 1, a ring buffer 142 is unnecessary.

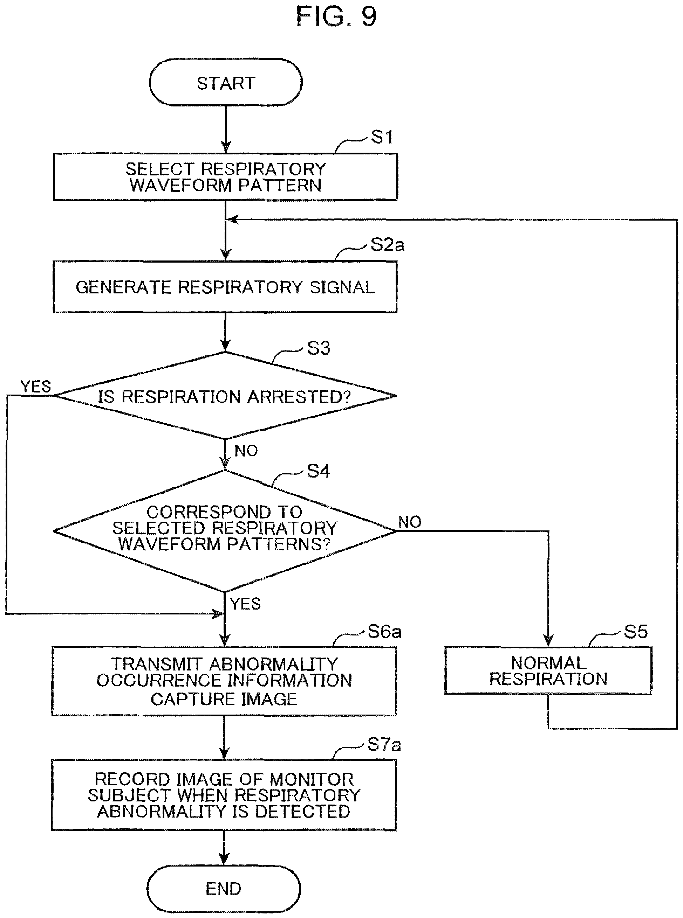

Respiratory abnormality detection and image recording to be performed by a monitor-subject monitoring system MS according to modification 1 will be described. FIG. 9 is a flowchart describing the respiratory abnormality detection and the image recording. The flowchart shown in FIG. 9 differs from the flowchart shown in FIG. 7 in the following points.

Modification 1 performs step S2a instead of step S2. In step S2a, the respiratory abnormality detection unit 11 generates the respiratory signal of the monitor subject Ob-1. This process is the same as generation of the respiratory signal of step S2.

Modification 1 performs step S6a instead of step S6. In step S6a, an SU communication IF unit 16 transmits abnormality occurrence information to a management server device SV. This process is the same as the process of step S6. Furthermore, in step S6a, when the respiratory abnormality detection unit 11 determines respiratory arrest of the monitor subject Ob-1 (Yes in step S3), or when the respiratory abnormality detection unit 11 determines that the respiratory signal generated in step S2a corresponds to one of the respiratory waveform pattern P-C (sleep apnea syndrome), the respiratory waveform pattern P-F (hypopnea) and the respiratory waveform pattern P-I (Kussmaul respiration) (Yes in step S4), the imaging control unit 13 causes the imaging unit 12 to capture the image of the imaging target including the monitor subject Ob-1 (still image or moving image).

Modification 1 performs step S7a instead of step S7. In step S7a, the storage processing unit 143 causes the nonvolatile storage unit 141 to store the image captured in step S6a. Accordingly, the image of the monitor subject Ob-1 when the respiratory abnormality is detected is recorded.

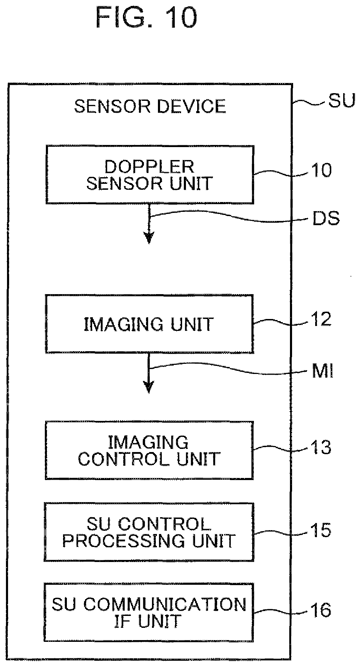

Modification 2 will be described. In modification 2, a management server device SV has a function of a living body monitoring device (respiratory abnormality detection device). FIG. 10 is a block diagram showing a configuration of a sensor device SU included in modification 2. FIG. 11 is a block diagram showing a configuration of the management server device SV included in modification 2. A difference from the sensor device SU and the server device SV included in the embodiment shown in FIG. 3 and FIG. 6 will be described.

In the embodiment, the respiratory abnormality detection unit 11 and the image recording unit 14 are included in the sensor device SU. In contrast, in modification 2, a respiratory abnormality detection unit 11 and an image recording unit 14 are included in the management server device SV. An SU control processing unit 15 orders an SU communication IF unit 16 to transmit a Doppler signal DS and a moving image MI to the management server device SV. The SU communication IF unit 16 changes the Doppler signal DS and the moving image MI into a communication signal that enables communication with an SV communication IF unit 34, and transmits the communication signal to the management server device SV.

In the embodiment, the Doppler sensor unit 10 functions as the acquisition unit that acquires the biological signal of the monitor subject Ob. In contrast, in modification 2, the SV communication IF unit 34 has a function of the acquisition unit. In the embodiment, the storage processing unit 143 has a function of, when a respiratory abnormality of the monitor subject Ob is detected, acquiring the image of the monitor subject Ob captured by the imaging unit 12 during a time period including the time when the respiratory abnormality is detected. In contrast, in modification 2, the SV communication IF unit 34 has this function.

### (Summary of Embodiment)

A respiratory abnormality detection device according to a first aspect of the present embodiment includes: an acquisition unit configured to acquire a biological signal of a monitor subject serving as a target to be monitored; a generation unit configured to generate a respiratory signal indicating a respiratory state of the monitor subject by using the biological signal acquired by the acquisition unit; a selection unit configured to select one or more respiratory waveform patterns from among a plurality of respiratory waveform patterns indicating a plurality of types of respiratory abnormalities by using a predetermined indicator serving as an indicator indicating a health condition of the monitor subject; and a determination unit configured to determine whether a waveform of the respiratory signal generated by the generation unit corresponds to one of the one or more respiratory waveform patterns selected by the selection unit, by using the waveform of the respiratory signal generated by the generation unit and the one or more respiratory waveform patterns selected by the selection unit.

It is known that there is a relationship between the indicator indicating the health condition of the monitor subject and the type of respiratory abnormality. An example will be described in which the indicator indicating the health condition is body weight. When body weight decreases significantly, a possibility of one of bradypnea, Cheyne-Stokes respiration, and ataxic respiration (Biot respiration) is high, and a possibility of other types of respiratory abnormalities is low. The respiratory abnormality detection device according to the first aspect of the present embodiment selects one or more respiratory waveform patterns from among the plurality of respiratory waveform patterns indicating the plurality of types of respiratory abnormalities by using the predetermined indicator serving as the indicator indicating the health condition of the monitor subject. Since this makes it possible to reduce the number of types of respiratory waveform patterns of respiratory abnormalities (since the types can be narrowed down), erroneous determination can be reduced in the determination of whether the waveform of the respiratory signal of the monitor subject corresponds to the respiratory waveform patterns of respiratory abnormalities. Therefore, the respiratory abnormality detection device according to the first aspect of the present embodiment can improve determination accuracy of whether respiration is abnormal and determination accuracy of the type of respiratory abnormality.

The predetermined indicator is at least one of vital data, dietary intake, and sleeping hours of the monitor subject. The vital data includes at least one of body temperature data, body weight data, and blood pressure data.

A respiratory abnormality detection method according to a second aspect of the present embodiment includes: an acquisition step of acquiring a biological signal of a monitor subject serving as a target to be monitored; a generation step of generating a respiratory signal indicating a respiratory state of the monitor subject by using the biological signal acquired in the acquisition step; a selection step of selecting one or more respiratory waveform patterns from among a plurality of respiratory waveform patterns indicating a plurality of types of respiratory abnormalities by using a predetermined indicator serving as an indicator indicating a health condition of the monitor subject; and a determination step of determining whether a waveform of the respiratory signal generated in the generation step corresponds to one of the one or more respiratory waveform patterns selected in the selection step, by using the waveform of the respiratory signal generated in the generation step and the one or more respiratory waveform patterns selected in the selection step.

The respiratory abnormality detection method according to the second aspect of the present embodiment defines the respiratory abnormality detection device according to the first aspect of the present embodiment from a viewpoint of a method, and has functions and effects similar to functions and effects of the respiratory abnormality detection device according to the first aspect of the present embodiment.

This application is based on Japanese Application No. 2016-128650 filed on June 29, 2016, the entire contents of which are incorporated herein by reference.

In order to describe the present invention, the present invention has been appropriately and fully described above by means of the embodiment with reference to the drawings, but it should be appreciated that one skilled in the art should be able to modify and/or improve the embodiment described above easily. Therefore, as long as the modification or improvement implemented by one skilled in the art does not depart from the scope of the claims, it is understood that the modification or improvement is included in the scope of the claims.

### Industrial Applicability

The present invention can provide the respiratory abnormality detection device and the respiratory abnormality detection method.

## Claims

1. A respiratory abnormality detection device comprising:
an acquisition unit configured to acquire a biological signal of a monitor subject serving as a target to be monitored;
a generation unit configured to generate a respiratory signal indicating a respiratory state of the monitor subject by using the biological signal acquired by the acquisition unit;
a selection unit configured to select one or more respiratory waveform patterns from among a plurality of respiratory waveform patterns indicating a plurality of types of respiratory abnormalities by using a predetermined indicator serving as an indicator indicating a health condition of the monitor subject; and
a determination unit configured to determine whether a waveform of the respiratory signal generated by the generation unit corresponds to one of the one or more respiratory waveform patterns selected by the selection unit, by using the waveform of the respiratory signal generated by the generation unit and the one or more respiratory waveform patterns selected by the selection unit.

2. The respiratory abnormality detection device according to claim 1, wherein the predetermined indicator is at least one of vital data, dietary intake, and sleeping hours of the monitor subject.

3. The respiratory abnormality detection device according to claim 2, wherein the vital data includes at least one of body temperature data, body weight data, and blood pressure data.

4. A respiratory abnormality detection method comprising:
an acquisition step of acquiring a biological signal of a monitor subject serving as a target to be monitored;
a generation step of generating a respiratory signal indicating a respiratory state of the monitor subject by using the biological signal acquired in the acquisition step;
a selection step of selecting one or more respiratory waveform patterns from among a plurality of respiratory waveform patterns indicating a plurality of types of respiratory abnormalities by using a predetermined indicator serving as an indicator indicating a health condition of the monitor subject; and
a determination step of determining whether a waveform of the respiratory signal generated in the generation step corresponds to one of the one or more respiratory waveform patterns selected in the selection step, by using the waveform of the respiratory signal generated in the generation step and the one or more respiratory waveform patterns selected in the selection step.
